# EUROPEAN PATENT APPLICATION

(11) **EP 4 464 303 A1**
(43) Date of publication of application: **20.11.2024**
(21) Application number: 24175149.4
(22) Date of filing: 10.05.2024
(51) Int. Cl.: A61K 6/90, C08L 5/04

(54) **BASE MATERIAL PASTE, DENTAL ALGINATE IMPRESSION MATERIAL, AND METHOD OF PREPARING DENTAL ALGINATE IMPRESSION MATERIAL**

(30) Priority: 12.05.2023 JP 2023079446
(71) Applicant: GC Corporation, Sunto-gun, Shizuoka 410-1307 (JP)
(72) Inventor: HIRANO, Ayumu, Itabashi-ku, Tokyo, 174-8585 (JP); ODA, Kotoka, Itabashi-ku, Tokyo, 174-8585 (JP)
(74) Representative: Müller-Boré & Partner Patentanwälte PartG mbB

(57) **Abstract**

A base material paste used for a dental alginate impression material is provided. The base material paste includes: an alginate; water; and an oily component that is liquid at normal temperature, a lipid component that is liquid at normal temperature, or both.

## Description

### TECHNICAL FIELD

The present invention relates to a base material paste, a dental alginate impression material, and a method of preparing dental alginate impression material.

### BACKGROUND ART

In dental treatment, an alginate impression material is widely used as an impression material for obtaining an impression of the oral cavity when preparing a prosthesis. As the alginate impression material, an alginate impression material including: a base material paste that includes an alginate and water; and a curing material paste that includes a gelling reagent and a poorly water-soluble liquid compound, is known (see, for example, Patent Documents 1 and 2).

### [Related-Art Documents]

### [Patent Documents]

[Patent Document 1] International Publication No. WO 2012/063618
[Patent Document 2] International Publication No. WO 2014/050028

### SUMMARY OF THE INVENTION

### [Problems to be Solved by the Invention]

However, in the conventional alginate impression material, the viscosity of the base material paste tends to decrease due to changes over time, and there is a problem with the storage stability of the base material paste.

An object of the present invention is to provide a base material paste with excellent storage stability.

### [Means for solving the problem]

One aspect of the present invention is a base material paste including used for a dental alginate impression material, the base material paste including: an alginate; water; and an oily component that is liquid at normal temperature, a lipid component that is liquid at normal temperature, or both.

### [Effects of the Invention]

According to one aspect of the present invention, a base material paste with excellent storage stability can be provided.

### DETAILED DESCRIPTION OF THE INVENTION

Hereinafter, embodiments for carrying out the present invention will be described in detail.

### [Base Material Paste]

A base material paste according to the present embodiment is used for a dental alginate impression material.

In the present specification, the dental alginate impression material is a dental preparation for obtaining an impression by utilizing the fact that when a water-soluble alginate reacts with gypsum, insoluble calcium alginate is produced and cured. The base material paste is a pasty material constituting the base material of the dental alginate impression material.

The base material paste includes an alginate, water, an oily component that is liquid at normal temperature, and/or a lipid component that is liquid at normal temperature.

Examples of the alginate include alkali metal salts of alginate such as sodium alginate and potassium alginate, ammonium salts of alginate such as ammonium alginate and triethanolamine alginate, and the like. Among them, alkali metal salts of alginate are preferable, and potassium alginate is more preferable, because of their availability; ease of handling; physical properties of the cured body of the dental alginate impression material according to the present embodiment described later; and so on.

The alginate may be used alone or in combination of two or more.

The viscosity of the 1 mass% aqueous solution of the alginate at 20°C is preferably 0.001 Pa·s or more and 0.5 Pa·s or less, and more preferably 0.1 Pa·s or more and 0.4 Pa·s or less. When the viscosity of the 1 mass% aqueous solution of the alginate at 20°C is 0.001 Pa·s or more, the impression is less likely to be damaged when the dental impression material using the base material paste according to the present embodiment is used in combination with a conventional alginate impression material.

When the viscosity of the 1 mass% aqueous solution of the alginate at 20°C is 0.5 Pa·s or less, the fluidity of the mixture of the base material paste and the curing material paste is higher. Accordingly, it is possible to improve the accuracy of the impression when the base material paste according to the present embodiment is used in combination with the alginate impression material.

The content of the alginate in the base material paste is not particularly limited, but is, for example, 0.5 mass% or more and 30 mass% or less, preferably 1 mass% or more and 20 mass% or less, and more preferably 3 mass% or more and 15 mass% or less.

When the content of the alginate in the base material paste is 0.5 mass% or more, the impression is less likely to be damaged when the alginate impression material (hereinafter sometimes referred to as a dental impression material) using the base material paste according to the present embodiment is used in combination with the conventional alginate impression material. When the content is 30 mass% or less, it is possible to improve the accuracy of the impression when the dental impression material using the base material paste according to the present embodiment is used in combination with the conventional alginate impression material.

As the water, for example, ion-exchange water, distilled water, or the like may be used, and water sterilized with sodium hypochlorite or the like may be used.

The content of water in the base material paste is not particularly limited, but is, for example, 75 mass% or more and 99 mass% or less, preferably 80 mass% or more and 97 mass% or less, and more preferably 85 mass% or more and 95 mass% or less.

The oily component that is liquid at normal temperature (hereinafter sometimes referred to as an oily component) is an oily liquid within the normal temperature range (for example, within the range of 20°C±15°C). The lipid component that is liquid at normal temperature (hereinafter sometimes referred to as a lipid component) is a liquid containing lipid within the normal temperature range (for example, within the range of 20°C±15°C).

The melting point of the oily component and/or the lipid component is preferably, but not particularly limited to, 25°C or less, more preferably 21°C or less, and more preferably 15°C or less. When the melting point of the oily component and/or the lipid component is 25°C or less, the oily component and/or the lipid component can remain in a liquid state at normal temperature.

Examples of the oily component that is liquid at normal temperature include hydrocarbon compounds, fatty acids, oils and fats, and the like. Examples of the lipid component that is liquid at normal temperature includes phospholipids and the like. The oily component and/or the lipid component may be used alone or in combination of two or more.

Examples of the hydrocarbon compound include liquid paraffin and the like.

Examples of the fatty acid include oleic acid, linoleic acid, linolenic acid, and the like.

Examples of the oil and fat include vegetable oils such as linseed oil, soybean oil, cottonseed oil, olive oil, castor oil, corn oil, and the like.

Examples of the phospholipid include soybean-derived lecithin (soybean lecithin), and the like.

The content of the oily component and/or the lipid component in the base material paste is not particularly limited, but is, for example, preferably 0.5 mass% or more and 3.5 mass% or less, more preferably 1 mass% or more and 3 mass% or less, and more preferably 1.5 mass% or more and 2.5 mass% or less. When the content of the oily component and/or the lipid component in the base material paste is 0.5 mass% or more, the effect of minimizing viscosity reduction is easily obtained. When the content is 3.5 mass% or less, separation of the paste is less likely to occur.

The base material paste may contain other components as long as the object of the present invention is not impaired. Examples of the other components contained in the base material paste may include fillers, colorants, preservatives, disinfectants, fragrances, pH adjusters, surfactants, curing retarders, and the like.

Examples of materials constituting the filler include clay minerals such as diatomaceous earth, talc, and smectite, and inorganic oxides such as silica, alumina, and the like. The filler may be used alone or in combination of two or more.

Examples of the surfactant include polyoxyethylene alkyl ether and the like. The surfactant may be used alone or in combination of two or more.

Examples of the curing retarder include phosphates such as trisodium phosphate and tripotassium phosphate, and condensed phosphates such as sodium pyrophosphate and potassium pyrophosphate, and the like. The curing retarder may be used alone or in combination of two or more.

The viscosity of the base material paste at 20°C is preferably 50,000 mPa·s or more and 110,000 mPa·s or less, more preferably 75,000 mPa·s or more and 105,000 mPa·s or less, and more preferably 80,000 mPa·s or more and 100,000 mPa·s or less.

When the viscosity of the base material paste at 20°C is 50,000 mPa·s or more, the mixability is improved even in the base material paste after storage. When the viscosity of the base material paste at 20°C is 110,000 mPa·s or less, the strength of the cured body of the dental impression material is improved even in the base material paste after storage.

The base material paste according to the present embodiment has a lower viscosity than conventional pasty alginate impression materials, and can be used in a combined impression method.

As described above, the base material paste according to the present embodiment includes the alginate, water, and the oily component that is liquid at normal temperature and/or a lipid component that is liquid at normal temperature. Accordingly, the viscosity reduction of the base material paste due to changes over time can be minimized and a base material paste with high storage stability can be obtained.

The base material paste according to the present embodiment can have a lower viscosity than the conventional pasty alginate impression materials, and can minimize the viscosity reduction while maintaining a low viscosity. Therefore, the base material paste according to the present embodiment can be used for the combined impression method (a method of combining two uncured materials).

### [Dental Alginate Impression Material]

The dental alginate impression material according to the present embodiment includes a base material paste and a curing material paste.

As the base material paste, the base material paste according to the present embodiment (the base material paste including the alginate, water, the oily component that is liquid at normal temperature, and/or the lipid component that is liquid at normal temperature) is used.

As the curing material paste, the curing material paste described above (the curing material paste that includes a gelling reagent and a poorly water-soluble liquid compound) is used.

The content of the alginate in the dental alginate impression material is not particularly limited, but is, for example, 0.3 mass% or more and 20 mass% or less, preferably 0.6 or more and 13 mass% or less, more preferably 2 or more and 10 mass% or less.

When the content of the alginate in the dental alginate impression material according to the present embodiment is 0.3 mass% or more, the impression is less likely to be damaged when the dental alginate impression material according to the present embodiment is used in combination with the conventional alginate impression material. When the content of the alginate in the dental alginate impression material according to the present embodiment is 20 mass% or less, it is possible to improve the accuracy of the impression when the dental alginate impression material according to the present embodiment is used in combination with the conventional alginate impression material.

The content of water in the dental alginate impression material according to the present embodiment is not particularly limited, but is, for example, 40 mass% or more and 75 mass% or less, preferably 45 mass% or more and 70 mass% or less, and more preferably 50 mass% or more and 65 mass% or less.

The content of the oily component and/or the lipid component in the dental alginate impression material according to the present embodiment is not particularly limited, but is, for example, 0.3 mass% or more and 2.5 mass% or less, preferably 0.6 mass% or more and 2 mass% or less, more preferably 1 mass% or more and 1.5 mass% or less.

When the content of the oily component and/or the lipid component in the dental alginate impression material is 0.3 mass% or more, the viscosity reduction of the base material paste is minimized even after storage, and the storage stability of the base material paste can be improved. When the content of the oily component and/or the lipid component in the base material paste is 2.5 mass% or less, the viscosity reduction of the base material paste can be minimized while controlling the viscosity of the base material paste to be low.

### [Curing Material Paste]

The curing material paste is a material that is mixed with the base material paste to cure the dental alginate impression material.

The curing material paste includes a gelling reagent and a poorly water-soluble liquid compound.

As the gelling reagent, compounds of divalent or higher valent metals may be used.

The compound of divalent or higher valent metals is not particularly limited, but is, for example, divalent or higher-valent metals, their salts, oxides, hydroxides, and the like. The compounds may be used alone or in combination of two or more.

Examples of the divalent or higher-valent metal include calcium, magnesium, zinc, aluminum, iron, titanium, zirconium, tin, and the like.

Examples of the salt of the divalent or higher-valent metal include calcium sulfate dihydrate (CaSO₄·2H₂O), calcium sulfate hemihydrate (CaSO₄·1/2H₂O), anhydrous calcium sulfate (CaSO₄), and the like.

Examples of the oxide of the divalent or higher-valent metal include calcium oxide, magnesium oxide, zinc oxide, titanium oxide, zirconium oxide, tin oxide, and the like.

Examples of the hydroxide of the divalent or higher-valent metal include calcium hydroxide, magnesium hydroxide, zinc hydroxide, aluminum hydroxide, iron hydroxide, and the like.

The content of the gelling reagent in the curing material paste is not particularly limited, but is preferably 10 mass% or more and 60 mass% or less. When the content of the gelling reagent in the curing material paste is 10 mass% or more, the strength of the cured body of the dental alginate impression material using the curing material paste is improved. When the content is 60 mass% or less, the time available to operate the dental impression material is extended.

The poorly water-soluble liquid compound is not particularly limited as long as the gelling reagent can be made into a paste, but examples of the poorly water-soluble liquid compound include hydrocarbon compounds, aliphatic alcohols, aromatic alcohols, fatty acids, fatty acid esters, silicone oils, and the like. These compounds may be used alone, or in combination of two or more. The poorly water-soluble liquid compound may be the same as the oily component and/or the lipid component used in the base material paste described above.

In the present specification, "poorly water-soluble" means that the solubility in 100 g of water at 20°C is 5 g or less.

Examples of the hydrocarbon compound include aliphatic chain hydrocarbon compounds such as hexane, heptane, octane, nonane, decane, undecane, dodecane, tridecane, tetradecane, pentadecane, kerosene, 2,7-dimethyloctane, and 1-octene; alicyclic hydrocarbon compounds such as cycloheptane and cyclononane; liquid paraffin; and the like.

Examples of the aliphatic alcohol include saturated aliphatic alcohols such as 1-hexanol and 1-octanol; unsaturated aliphatic alcohols such as citronellol and oleyl alcohol; and the like.

Examples of the aromatic alcohol include benzyl alcohol, m-cresol, and the like.

Examples of the fatty acids include saturated fatty acids such as hexanoic acid and octanoic acid; unsaturated fatty acids such as oleic acid and linoleic acid; and the like.

Examples of the fatty acid ester include ethyl octanoate, butyl phthalate, glyceride oleate, olive oil, sesame oil, liver oil, whale oil, and the like.

Examples of the silicone oil include polydimethylsiloxane, polymethylphenylsiloxane, polymethylhydrogensiloxane, polyphenylhydrogensiloxane, and the like.

The content of the poorly water-soluble liquid compound in the curing material paste is not particularly limited, but is, for example, preferably 10 mass% or more and 50 mass% or less, and more preferably 15 mass% or more and 30 mass% or less. When the content of the poorly water-soluble liquid compound in the curing material paste is 10 mass% or more, the operability of the dental alginate impression material according to the present embodiment using the curing material paste is improved. When the content is 50 mass% or less, the strength of the cured body of the dental alginate impression material is improved.

The curing material paste preferably further contains polybutene. Accordingly, the curing property of the dental alginate impression material according to the present embodiment is improved, and the time available to operate the dental alginate impression material is extended.

The polybutene is preferably liquid. The polybutene may be synthesized by copolymerizing isobutene and 1-butene. The number average molecular weight of the polybutene is preferably 300 to 4,000.

The content of the polybutene in the curing material paste is preferably 0.5 mass% or more and 30 mass% or less, and more preferably 1 mass% or more and 20 mass% or less. When the content of the polybutene in the curing material paste is 0.5 mass% or more and 30 mass% or less, the operability of the dental alginate impression material according to the present embodiment is improved.

The curing material paste may further contain fillers, curing retarders, curing modifiers, surfactants, colorants, preservatives, disinfectants, fragrances, pH adjusters, and the like.

Examples of the materials constituting the filler include clay minerals such as diatomaceous earth, talc, and smectite, and inorganic oxides such as silica, alumina, and the like. The filler may be used alone or in combination of two or more.

Examples of the curing retarder include phosphates such as trisodium phosphate and tripotassium phosphate, and condensed phosphates such as sodium pyrophosphate and potassium pyrophosphate. The curing retarder may be used alone or in combination of two or more.

Examples of the curing modifier include potassium titanium fluoride, potassium silicofluoride, and the like. The curing modifier may be used alone or in combination of two or more.

Examples of the surfactant include polyoxyethylene alkyl ether and the like. The surfactant may be used alone or in combination of two or more.

Examples of the consistency of the curing material paste is not particularly limited, but is preferably 30 mm or more. When the consistency of the curing material paste is 30 mm or more, the fluidity of the mixture of the base material paste and the curing material paste is higher. Accordingly, it is possible to improve the accuracy of the impression when the dental alginate impression material according to the present embodiment is used in combination with the conventional alginate impression material.

As described above, the dental alginate impression material according to the present embodiment includes the base material paste according to the present embodiment and the curing material paste, so that the effect of the base material paste according to the present embodiment can be obtained. That is, in the dental alginate impression material according to the present embodiment, because the viscosity reduction of the included base material paste due to changes over time is minimized and the storage stability of the base material paste is high, the reduction in the mixability of the dental alginate impression material can be minimized when the base material paste is used. Therefore, in the dental alginate impression material according to the present embodiment, the impression is less likely to be damaged, and the accuracy of the impression is improved.

Further, in the dental alginate impression material according to the present embodiment, the included base material paste has a lower viscosity than the conventional pasty alginate impression materials, and can minimize the viscosity reduction while maintaining a low viscosity. Therefore, the dental alginate impression material according to the present embodiment can be used for the combined impression method.

### [Method of Preparing Dental Alginate Impression Material]

A method of preparing a dental alginate impression material according to the present embodiment is a method of preparing the dental alginate impression material by mixing the base material paste and the curing material paste described above.

As the method of preparing the dental alginate impression material, for example, the base material paste and the curing material paste filled in a separated packaging container are extruded using a manual or electric extrusion apparatus, and passed through a nozzle to prepare a mixture. The obtained mixture can be applied to teeth as the dental alginate impression material.

Examples of the packaging container include resin or metal molded products, resin or metal bags, and the like.

The mass ratio of the base material paste to the curing material paste in mixing the base material paste and the curing material paste is not particularly limited, but is, for example, 1 to 5, preferably 1 to 4, and more preferably 1 to 3.

In the method of preparing the dental alginate impression material according to the present embodiment, the dental alginate impression material described above is obtained by mixing the base material paste described above and the curing material paste, so that the effect of the base material paste according to the present embodiment can be obtained.

Specifically, in the method of preparing the dental alginate impression material according to the present embodiment, because the viscosity reduction of the base material paste included in the obtained dental alginate impression material due to changes over time is minimized and the storage stability of the base material paste is high, the reduction in the mixability of the dental alginate impression material can be minimized when the base material paste is used. Therefore, by using the method of preparing the dental alginate impression material according to the present embodiment, the impression of the obtained dental alginate impression material is less likely to be damaged, and the accuracy of the impression is improved.

Further, in the dental alginate impression material obtained by the method of preparing the dental alginate impression material according to the present embodiment, the included base material paste has a lower viscosity than the conventional pasty alginate impression materials, and can minimize the viscosity reduction while maintaining a low viscosity. Therefore, by using the method of preparing the dental alginate impression material according to the present embodiment, the obtained dental alginate impression material can be used for the combined impression method.

### [Combined Impression Material]

As a combined impression material according to the present embodiment, the dental alginate impression material according to the present embodiment described above can be used. In the present specification, the combined impression material is a material for an impression by the combined impression method (a method of combining two uncured materials).

The combined impression material according to the present embodiment is used in combination with other alginate impression materials. More specifically, after the combined impression material according to the present embodiment is applied to an impression object such as a tooth, the other alginate impression material is applied.

The other alginate impression material is not particularly limited, but for example, an alginate impression material used in the agar-alginate combined impression method may be used.

The other alginate impression material may be a powder type, or may be a paste type that includes a base material paste containing an alginate and water and a curing material paste containing a gelling reagent and a poorly water-soluble liquid compound.

The mass ratio of the combined impression material according to the present embodiment to the other alginate impression material is not particularly limited.

When the paste type alginate impression material is used as the other alginate impression material, the alginate impression material is the same as the dental alginate impression material according to the present embodiment except that the consistency of the mixture of the base material paste and the curing material paste are less than 36 mm.

As described above, the combined impression material according to the present embodiment has the effect of the dental alginate impression material according to the present embodiment, by using the dental alginate impression material according to the present embodiment. That is, in the combined impression material according to the present embodiment, because the storage stability of the included base material paste is high (resistant to deterioration even after storage), the reduction in the mixability of the dental alginate impression material can be minimized when the base material paste is used, and the obtained impression is less likely to be damaged.

In the combined impression material according to the present embodiment, the base material paste included in the dental alginate impression material has a lower viscosity than the conventional pasty alginate impression materials, and can minimize the viscosity reduction while maintaining a low viscosity. Therefore, in the combined impression material according to the present embodiment, it is possible to improve the accuracy of the impression using the combined impression method.

### Examples

Hereinafter, the present invention will be described with reference to a further example. In the following, unitless numbers, "parts" or "%" are mass standards unless otherwise specified. Examples and comparative examples were evaluated by the following tests.

### [Example 1]

### <Preparation of Base Material Paste>

A base material paste was prepared by mixing 7 parts of potassium alginate and 3 parts of sodium alginate as alginates, and 2 parts of linseed oil as an oily component that is liquid at normal temperature, and adding distilled water so that the total volume was 100 parts. The composition of the base material paste is presented in Table 1.

### <Preparation of Curing Material Paste>

A curing material paste was prepared by mixing 45 parts of anhydrous calcium sulfate, 23 parts of liquid paraffin, 12.5 parts of silica, 6 parts of magnesium hydroxide, 4 parts of polybutene, 3 parts of potassium titanium fluoride, 3 parts of polyoxyethylene alkyl ether, 2 parts of zinc oxide, and 1.5 parts of tripotassium phosphate. The composition of the curing material paste is presented in Table 2.

### [Example 2]

The base material paste and the curing material paste were prepared in the same manner as in Example 1, except that 2 parts of liquid paraffin were mixed as the oily component that is liquid at normal temperature in the preparation of the base material paste. The composition of the base material paste is presented in Table 1.

### [Example 3]

The base material paste and the curing material paste were prepared in the same manner as in Example 1, except that 2 parts of oleic acid were mixed as the oily component that is liquid at normal temperature in the preparation of the base material paste. The composition of the base material paste is presented in Table 1.

### [Example 4]

The base material paste and the curing material paste were prepared in the same manner as in Example 1, except that 2 parts of soybean lecithin were mixed as a lipid component that is liquid at normal temperature in the preparation of the base material paste. The composition of the base material paste is presented in Table 1.

### [Comparative Example 1]

The base material paste and the curing material paste were prepared in the same manner as in Example 1, except that neither an oily component that is liquid at normal temperature nor a lipid component that is liquid at normal temperature was mixed in the preparation of the base material paste. The composition of the base material paste is presented in Table 1.

### [Comparative Example 2]

The base material paste and the curing material paste were prepared in the same manner as in Example 1, except that 2 parts of palm oil were mixed as an oily component that is solid at normal temperature in the preparation of the base material paste. The composition of the base material paste is presented in Table 1.

### [Comparative Example 3]

The base material paste and the curing material paste were prepared in the same manner as in Example 1, except that 2 parts of vaseline were mixed as an oily component that is solid at normal temperature in the preparation of the base material paste. The composition of the base material paste is presented in Table 1.

### [Comparative Example 4]

The base material paste and the curing material paste were prepared in the same manner as in Example 1, except that 2 parts of palmitic acid were mixed as an oily component that is solid at normal temperature in the preparation of the base material paste. The composition of the base material paste is presented in Table 1.

### [Comparative Example 5]

The base material paste and the curing material paste were prepared in the same manner as in Example 1, except that 2 parts of hydrogenated lecithin were mixed as a lipid component that is solid at normal temperature in the preparation of the base material paste. The composition of the base material paste is presented in Table 1.

### <Storage Stability>

The viscosity of the prepared base material paste was measured at 23°C using a viscometer (E-type viscometer RE-85, manufactured by Toki Sangyo Co., Ltd.) under the conditions of a cone angle of 3° and a shear rate 2 (1/sec). The base material paste was then filled into a 50 mL clear glass bottle, sealed, and stored in a thermostatic bath at 45°C for 7 days. After storage, the base material paste was stored in a thermostatic bath at 23°C for 1 hour, and the viscosity was measured again. The viscosity values before and after storage were used to calculate the viscosity reduction rate, according to the following formula. The evaluation criteria were as follows. The viscosity reduction rates for each of the examples and comparison examples are presented in Table 1. Viscosity reduction rate (%) = 100 - (viscosity after storage/viscosity before storage)x100

### [Evaluation Criteria]

Good: Viscosity reduction rate was less than 45%
Poor: Viscosity reduction rate was 45% or more

### <Mixability After Storage>

The base material paste after being stored for 1 hour in the above storage stability measurement and the curing material paste were filled into a mixing cartridge at a mass ratio of 2:1, and then the paste was extruded and mixed. The mixability after storage was evaluated from the appearance of the paste (the dental alginate impression material) discharged from the nozzle. The evaluation criteria were as follows. The mixability after storage for each of the examples and comparison examples are presented in Table 1.

### [Evaluation Criteria]

Good: The base material paste and the curing material paste were discharged in a uniformly mixed state.
Poor: The base material paste was discharged earlier than the curing material paste, and the mixing was uneven.

**[Table 1]**

| | EXAMPLE | | | | COMPARATIVE EXAMPLE | | | | |
|---|---|---|---|---|---|---|---|---|---|
| COMPOSITION RATIO (MASS %) | 1 | 2 | 3 | 4 | 1 | 2 | 3 | 4 | 5 |
| POTASSIUM ALGINATE | 7 | 7 | 7 | 7 | 7 | 7 | 7 | 7 | 7 |
| SODIUM ALGINATE | 3 | 3 | 3 | 3 | 3 | 3 | 3 | 3 | 3 |
| LINSEED OIL (LIQUID AT ROOM TEMPERATURE) | 2 | | | | | | | | |
| LIQUID PARAFFIN (LIQUID AT ROOM TEMPERATURE) | | 2 | | | | | | | |
| OLEIC ACID (LIQUID AT ROOM TEMPERATURE) | | | 2 | | | | | | |
| SOY LECITHIN (LIQUID AT ROOM TEMPERATURE) | | | | 2 | | | | | |
| COCONUT OIL (SOLID AT ROOM TEMPERATURE) | | | | | | 2 | | | |
| VASELINE (SOLID AT ROOM TEMPERATURE) | | | | | | | 2 | | |
| PALMITIC ACID (SOLID AT ROOM TEMPERATURE) | | | | | | | | 2 | |
| HYDROGENATED LECITHIN (SOLID AT ROOM TEMPERATURE) | | | | | | | | | 2 |
| DISTILLED WATER | 88 | 88 | 88 | 88 | 90 | 88 | 88 | 88 | 88 |
| VISCOSITY BEFORE STORAGE (mPa·s) | 85500 | 83000 | 87500 | 96000 | 81000 | 86500 | 95000 | 94500 | 83000 |
| VISCOSITY AFTER STORAGE (mPa·s) | 52500 | 50500 | 54500 | 61000 | 42300 | 43000 | 32000 | 47500 | 40600 |
| VISCOSITY REDUCTION RATE (%) | 38.6 | 39.2 | 37.7 | 36.5 | 47.8 | 50.3 | 66.3 | 49.7 | 51.1 |
| STORAGE STABILITY | GOOD | GOOD | GOOD | GOOD | POOR | POOR | POOR | POOR | POOR |
| MIXABILITY AFTER STORAGE | GOOD | GOOD | GOOD | GOOD | POOR | POOR | POOR | POOR | POOR |

**[Table 2]**

| COMPOSITION OF CURING MATERIAL PASTE | (PARTS) |
|---|---|
| ANHYDROUS CALCIUM SULFATE | 45 |
| MAGNESIUM HYDROXIDE | 6 |
| ZINC OXIDE | 2 |
| LIQUID PARAFFIN | 23 |
| POLYBUTENE | 4 |
| TRIPOTASSIUM PHOSPHATE | 1.5 |
| SODIUM PYROPHOSPHATE | 1 |
| POTASSIUM TITANIUM FLUORIDE | 3 |
| POLYOXYETHYLENE ALKYL ETHER | 3 |
| SILICA | 12.5 |
| TOTAL | 101 |

From Table 1, the base material pastes containing the alginate, water, and the oily component that is liquid at normal temperature or the lipid component that is liquid at normal temperature all exhibited good storage stability and mixability after storage (Examples 1 to 4).

In contrast, the base material pastes containing neither an oily component that is liquid at normal temperature nor a lipid component that is liquid at normal temperature exhibited poor storage stability and mixability after storage (Comparative Examples 1 to 5) .

From these results, the base material paste containing the alginate, water, and the oily component that is liquid at normal temperature or the lipid component that is liquid at normal temperature exhibited excellent storage stability, and the dental alginate impression material prepared by mixing the base material paste after storage and the curing material paste exhibited good mixability.

Although the embodiments of the present invention have been described above, the present invention is not limited to the specific embodiments, and various modifications and changes can be made within the scope of the invention described in the claims.

This patent application is based on and claims priority to Japanese Patent Application No. 2023-079446 filed on May 12, 2023, the entire contents of which are incorporated herein by reference.

## Claims

1. A base material paste used for a dental alginate impression material, the base material paste comprising:
an alginate;
water; and
an oily component that is liquid at normal temperature, a lipid component that is liquid at normal temperature, or both.

2. A dental alginate impression material comprising:
the base material paste of claim 1; and
a curing material paste that includes a gelling reagent and a poorly water-soluble liquid compound.

3. A method of preparing a dental alginate impression material, the method comprising:
mixing the base material paste of claim 1 and a curing material paste that includes a gelling reagent and a poorly water-soluble liquid compound.
